# EUROPEAN PATENT APPLICATION

(11) **EP 3 889 270 A2**
(43) Date of publication of application: **06.10.2021**
(21) Application number: 19891145.5
(22) Date of filing: 26.11.2019
(51) Int. Cl.: C12Q 1/68, C12N 15/11

(54) **DNA-CUTTING AGENT**

(30) Priority: 26.11.2018 RU 2018141534
(71) Applicant: Joint Stock Company "Biocad", St.Petersburg 198515 (RU)
(72) Inventor: SEVERINOV, Konstantin Viktorovich, Moscow, 119333 (RU); SHMAKOV, Sergey Anatolevich, Voskresensk, 140200 (RU); ARTAMONOVA, Daria Nikolaevna, Moscow, 121359 (RU); GORYANIN, Ignatiy Igorevich, Moscow, 117587 (RU); MUSHAROVA, Olga Sergeevna, Moscow, 123098 (RU); PISKUNOVA, Iuliia Valerevna, Moscow, 142784 (RU); FEDOROVA, Iana Vitalevna, Gatchina, 188301 (RU); ZYUBKO, Tatyana Igorevna, St. Petersburg, 197374 (RU); KHODORKOVSKIY, Mikhail Alekseevich, St.Petersburg, 195426 (RU); POBEGALOV, Georgii Evgenevich, St. Petersburg, 198205 (RU); ARSENIEV, Anatolii Nikolaevich, St. Petersburg, 195112 (RU); SELKOVA, Polina Anatolevna, Votkinsk, 427439 (RU); VASILEVA, Aleksandra Andreevna, St. Petersburg, 198334 (RU); ARTAMONOVA, Tatiana Olegovna, St. Petersburg, 199406 (RU); ABRAMOVA, Marina Viktorovna, St. Petersburg, 194021 (RU)
(74) Representative: Held, Stephan
(86) International application number: PCT/RU2019/050230
(87) International publication number: WO 2020/111984

(57) **Abstract**

The present invention describes a novel bacterial nuclease of the CRISPR-Cas9 system from the bacterium *Defluviimonas sp.* 20V17, as well as the use thereof to form strictly specific double-strand breaks in a DNA molecule. This nuclease has unusual properties and may be used as a tool for introducing modifications at strictly defined sites in the genomic DNA sequence of unicellular or multicellular organisms. Thus, the versatility of the available CRISPR-Cas9 systems is increased, which will enable the use of Cas9 nucleases from various organisms for cutting genomic or plasmid DNA in a larger number of specific sites and specific conditions.

Sequence listing

<110> JSC "BIOCAD"

<120> DNA-CUTTING AGENT

<130> 417254

<140> PCT/RU2019/050230
<141> 2019-11-26

<150> RU 2018141534
<151> 2018-11-26

<160> 2

<170> BiSSAP 1.3.6

<210> 1
<211> 1079
<212> PRT
<213> Defluviimonas

<220>
<223> protein homologous to Cas9

<400> 1
Met Tyr Arg Phe Ala Phe Asp Leu Gly Thr Asn Ser Leu Gly Trp Ala
1               5                   10                  15
Val Tyr Arg Leu Thr Asn Asp Thr Arg Pro Ala Ser Ile Glu Arg Thr
            20                  25                  30
Gly Val Arg Ile Phe Pro Asn Gly Arg Asp Pro Gln Ser Lys Glu Ser
        35                  40                  45
Asn Ala Ala Gly Arg Arg Met Pro Arg Gly Ala Arg Arg Arg Gln Asp
    50                  55                  60
Arg Ser Leu Gly Arg Arg Lys Arg Leu Leu Asp Asp Leu Ile Gly Phe
65                  70                  75                  80
Gly Leu Leu Pro Ser Asp Ala Asp Ala Arg Ser Ala Val Phe Ala Ala
                85                  90                  95
Asn Pro Ile Glu Ala Arg Ala Arg Ala Ala Arg Glu Gln Val Ala Leu
            100                 105                 110
Glu Gln Leu Gly Arg Ala Leu Trp His Met Ser Lys His Arg Gly Phe
        115                 120                 125
Lys Ser Asn Arg Arg Ala Asp Lys Asp Ala Asp Glu Lys Gly Lys Ile
    130                 135                 140
Ala Ile Ala Ser Ala Ala Leu Leu Glu Arg Leu Arg Ala Asp Gly His
145                 150                 155                 160
Pro Thr Tyr Gly Ala Phe Leu His Ala Arg Leu Val Arg Gly Glu Gly
                165                 170                 175
Thr Arg Ile Arg Pro Ala Gly Asp Gly Ala Lys Leu Ser Tyr Glu Phe
            180                 185                 190
Tyr Pro Thr Arg Ala Leu Leu Glu Ala Glu Phe Asp His Ile Trp Ala
        195                 200                 205
Ile Gln Ala Gly Phe His Pro Ser Leu Thr Glu Ala Met His Glu Arg
    210                 215                 220
Leu Arg Asp Thr Ile Phe Phe Gln Arg Pro Leu Arg Pro Val Arg Pro
225                 230                 235                 240
Gly Lys Cys Thr Phe Phe Pro Asp Gln Asp Arg Leu Pro Arg Trp His
                245                 250                 255
Pro Ala Ala Gln Glu Phe Leu Ile Leu Ser Gln Leu Asn His Leu Arg
            260                 265                 270
Ile Val Asp Asp Gln Gly Glu Gln Pro Leu Asp Ile Thr Ala Arg Asp

        275                 280                 285
Leu Val Ala Arg Thr Leu Met Ala Gly Thr Lys Leu Ser Trp Ser Gly
    290                 295                 300
Leu Lys Lys Thr Leu Lys Leu Pro Ser Gln Ala Glu Phe Asn Leu Glu
305                 310                 315                 320
Lys Gly Gly Leu Lys Glu Leu Ala Arg Asn Asp Val Ala Ala Arg Leu
                325                 330                 335
Leu Gly Asp Thr Lys Lys Pro Gly Pro Leu Ala Thr Leu Trp Pro Thr
            340                 345                 350
Leu Asp Ser Ala Thr Gln Glu Glu Ile Leu Trp Gln Ile Ser Glu Val
        355                 360                 365
Ala Asp Pro Glu Glu Leu Ile Cys Trp Leu Met Glu Arg Leu Gly Leu
    370                 375                 380
Ser Arg Glu Val Ala Glu Arg Val Glu Lys Ile Pro Leu Pro Asp Gly
385                 390                 395                 400
His Leu Arg Phe Cys Lys Thr Ala Thr Gln Ala Ile Val Glu Lys Leu
                405                 410                 415
Arg Cys Asp Val Ile Pro Tyr Asp Glu Ala Val Arg Arg Ala Pro Leu
            420                 425                 430
Leu Gly Gly Ala Gly Leu Asp His Ser Asp Phe Gln Ala Glu Glu Gly
        435                 440                 445
Val Asp Thr Leu Pro Pro Tyr Asn Arg Leu Pro Val Leu Gln Arg Met
    450                 455                 460
Ile Gly Asn Gly Thr Ser Asp Pro Lys Asp Thr Asp Leu Leu Arg Tyr
465                 470                 475                 480
Gly Arg Ile Thr Asn Pro Thr Val His Ile Ala Leu Gly Gln Phe Arg
                485                 490                 495
Arg Val Met Asn Ala Leu Ile Ala Glu Tyr Gly Lys Pro Ala Gln Val
            500                 505                 510
Val Ile Glu Ala Thr Arg Asp Met Ala Lys Ser Ala Glu Glu Leu Asn
        515                 520                 525
Lys Ile Glu Lys Thr Ile Arg Asp Asn Glu Lys Arg Asn Asp Arg Trp
    530                 535                 540
Arg Ala Glu Leu Gln Lys Ala Gly Leu Leu Val Glu Gly Ala Arg Ile
545                 550                 555                 560
Gly Asp Arg Phe Leu Arg Met Arg Leu Trp Glu Glu Ile Gly Arg Gly
                565                 570                 575
Pro Ala Asp Arg Leu Cys Pro Tyr Thr Gly Arg Pro Ile Ala Leu His
            580                 585                 590
Gln Leu His Ser Asp Glu Ile Glu Ile Asp His Ile Leu Pro Phe Glu
        595                 600                 605
Gln Thr Phe Asp Asp Ser Pro Ala Asn Lys Ser Leu Cys Phe Arg Asp
    610                 615                 620
Ala Asn Arg Arg Lys Gly Lys Leu Ser Pro Ala Ala Ala Ala Asp Arg
625                 630                 635                 640
Gln Pro Asp Phe Phe Asp Leu Ala Ala Ile Ile Gly Arg Thr Lys His
                645                 650                 655
Leu Pro Ala Asn Lys Ala Trp Arg Phe Leu Pro Gly Ala Met Glu Lys
            660                 665                 670
Trp Glu Glu Thr Arg Gly Phe Glu Asp Arg Gln Leu Asn Ala Thr Gly
        675                 680                 685
Tyr Leu Ala Arg Val Val Arg Ala Tyr Thr Glu Ala Leu Phe Pro Lys
    690                 695                 700
Thr Asp Ala Glu Gly Lys Arg Arg Ser His Val Trp Val Leu Pro Gly
705                 710                 715                 720
Arg Met Thr Ala Met Leu Arg His Arg Trp Gly Leu Asn Leu Gly Asp
                725                 730                 735
His Asn Arg Lys Ser Arg Asp Asp His Arg His His Ala Ile Asp Ala
            740                 745                 750
Ala Val Val Gly Val Ile Asp Arg Arg Met Val Gln Val Leu Gln Thr
        755                 760                 765
His Ala Arg Asn Leu Gly Val Glu Arg Leu Asp Arg Val Leu Pro Ala
    770                 775                 780

Pro Pro Glu Pro Phe Glu Gly Phe Arg Asp Ala Val Leu Ala Ala Val
785                 790                 795                 800
Glu Lys Val Asn Val Ser His Arg Ala Gln His Gly Ser Ile Asp Pro
                805                 810                 815
Thr Asp Pro Ser Gln Thr Ser Gly Arg Leu His Glu Asp Thr Val Phe
            820                 825                 830
Gly Leu Val Arg Asp Val Pro Glu Asn Gln Ala Glu Arg Thr Ile Gly
        835                 840                 845
Asn Val Val Val Arg Lys Pro Ile Thr Gly Leu Thr Glu Lys Glu Ile
    850                 855                 860
Gly Gln Val Arg Asp Val Lys Met Arg Leu Ser Leu Gln Asp Ala Thr
865                 870                 875                 880
Ser Leu Ala Arg Asp Lys Ser Leu Pro Glu Ala Glu Arg Lys Lys Arg
                885                 890                 895
Leu Pro Glu Ala Leu Ala Lys Trp Val Gln Glu Thr Gly His Arg Lys
            900                 905                 910
Leu Arg Ile Leu Lys Pro Glu Ala Gly Val Arg Pro Val His Asp Arg
        915                 920                 925
Asp Gly Lys Pro Tyr Lys Trp Leu Val Pro Gly Glu Ile Ser Trp Leu
    930                 935                 940
Asp Ile Leu Glu Ala Pro Asp Gly Thr Trp Phe Gln His Ala Thr Asp
945                 950                 955                 960
Ile Trp Ala Ala Asn Ser Gly Gly Ala Glu Pro Trp Asn Val Ala His
                965                 970                 975
Pro Ser Ala Arg Phe Ile Met Arg Val His Lys Asn Asp Thr Ile Gln
            980                 985                 990
Val Phe Asp Trp Asp Asp Arg Asn Lys Cys Val Val Glu Gly Ser Asn
        995                 1000                1005
Gln Ile Lys Arg Ile Val Arg Leu Glu Pro Ser Ala Asn Arg Met Arg
    1010                1015                1020
Leu Val Gly Ile Asn Asp Ala Gly Lys Phe Asp Glu Arg His Asn Glu
1025                1030                1035                1040
Pro Asn Asp Pro Phe Arg Trp Asp Leu Ala Thr Ile Ser Lys Leu Lys
                1045                1050                1055
Leu Arg Arg Ala Arg Arg Val Arg Ile Asp Glu Leu Gly Arg Val His
            1060                1065                1070
Thr Ile Pro His Gly Thr Val
        1075

<210> 2
<211> 3240
<212> DNA
<213> Defluviimonas

<400> 2
atgtatcgtt tcgctttcga cctcggaacc aactcccttg gctgggccgt ctatcgcttg      60

accaatgaca cgcggcccgc ttccattgaa aggacagggg tgaggatctt cccaaacggt     120

cgggatccac agtcgaagga gtcgaatgcc gctggccgca gaatgccgcg cggcgcaagg     180

cggaggcagg atcgaagcct gggacgaagg aagcggctgc tcgatgacct gatcgggttt     240

ggcctgctgc catcagatgc agatgcccgc agcgcggtct ttgccgcaaa tcccatcgaa     300

gcacgggctc gggccgcgcg cgaacaggtg gctctggaac aacttggccg ggcactctgg     360

cacatgtcga aacatcgcgg tttcaagagc aaccgccgcg ccgacaagga tgcggatgaa     420

aagggcaaga tcgcgatcgc ctcggccgcc cttctagagc gactgagggc agacgggcac     480

ccgacatacg gcgcctttct gcacgcgcgc ctcgtccgag gtgaaggaac gcgcatacgc     540

ccggcaggcg acggcgcaaa gctgtcctac gaattctacc cgacacgcgc tcttctggag 600

gccgagttcg atcacatctg ggccatccaa gcgggcttcc acccgtccct gaccgaagcg 660

atgcacgagc ggctgcgcga caccatcttc tttcagcgcc ccctgcgccc cgtccgtccg 720

gggaaatgca cattcttccc cgatcaggac cggctgccgc gctggcaccc ggccgcacag 780

gagtttctga tcctgtcaca gctcaaccat ttgcgcatcg tcgacgatca aggcgaacag 840

ccactggaca tcaccgcgcg ggatcttgtc gcgcgtacgt tgatggctgg aacaaaactg 900

tcatggtcgg gcctgaaaaa gaccctgaag ctgccgtcac aggccgagtt caacctcgaa 960

aagggcggcc tgaaggagct tgcacggaat gacgtggcgg cacgactgct gggcgatacg 1020

aagaaacccg gaccgctggc gacgctctgg ccgaccctcg acagcgccac gcaagaggaa 1080

atcctgtggc agatatcgga ggtcgcagac ccggaagagc tcatctgctg gcttatggaa 1140

aggctcgggc tttctcgcga ggtggcggaa agggtcgaga agatcccact gccggacggt 1200

cacctgcgtt tctgcaagac agcgacgcag gcgattgtcg aaaaactgcg ctgcgacgtc 1260

attccctatg acgaagccgt ccggcgcgcg cctttgcttg gcggggcggg gctggaccac 1320

tccgattttc aggccgaaga aggcgtcgat accctgccgc cctacaaccg gctgcccgtt 1380

ctacagcgca tgatcggcaa cggaaccagc gatccgaaag acacggacct gctgcgctat 1440

ggccggatca ccaacccgac cgtgcacatc gccttgggcc agttccggcg cgtgatgaat 1500

gcgctgatcg cagaatacgg taagcctgcg caggtcgtca tcgaggccac gcgagacatg 1560

gccaagtcag ccgaggaact gaacaagatc gagaaaacca tccgcgacaa cgaaaaacgc 1620

aatgatcgct ggcgtgccga gcttcagaaa gcggggctgc tggtggaagg cgcgcggatc 1680

ggcgaccggt tcttgaggat gcgcctatgg gaggagattg gcagaggccc ggcagatcgg 1740

ctctgtccct acaccggtcg ccccatcgcg ctgcatcagc tgcattccga cgagatcgag 1800

atcgatcata tcctgccctt cgagcaaacc ttcgacgaca gtccagccaa caagagcctg 1860

tgtttccgcg atgccaaccg ccgcaagggc aagctgtcgc cggctgcagc ggccgaccgg 1920

cagccagact tcttcgatct ggctgccatc atcgggcgca cgaaacacct gcccgccaac 1980

aaggcctggc ggtttctgcc cggtgccatg gaaaaatggg aagagacgcg cggtttcgag 2040

gatcgccagt tgaacgccac cggctatctg gcccgagttg tccgcgccta taccgaggcg 2100

ctgttcccga aaaccgatgc cgagggcaag cgacgcagcc atgtctgggt cctgcccggt 2160

cgcatgaccg cgatgttgcg gcaccgctgg ggtctgaacc tgggcgatca caaccgtaag 2220

tcacgcgacg atcaccgcca ccatgcgatc gacgccgccg tggtgggcgt gatcgaccgg 2280

cggatggtgc aggtgttgca gacccatgcg cggaacctgg gggtggagcg gctggaccgc 2340

gtgttacccg cgccgccaga acccttcgaa ggtttccgcg acgccgtgct ggctgccgtc 2400

gaaaaggtca acgtcagcca tcgtgcgcaa cacggcagca tcgacccaac cgatccatcg 2460

cagaccagcg gcaggctgca tgaggataca gtctttggtc ttgtgcggga tgtgccagag 2520

aaccaggccg agcgcaccat cggcaatgtc gttgtgcgca aacccataac cggcttgacc 2580

gaaaaggaaa tcggacaggt gcgcgacgtc aagatgcgcc tcagtctgca ggatgccacc 2640

agtttagccc gcgacaaatc cttgccggag gcagagcgca agaagcgcct gcccgaggcg 2700

ctggcgaaat gggttcagga aaccggccac cgcaaactgc gtatcctgaa gcccgaagct 2760

ggcgttcgcc ccgttcatga ccgtgatggc aagccctaca aatggctggt gcccggtgaa 2820

atctcctggc tcgacatcct agaagcgcct gacggcacat ggtttcagca cgccacagac 2880

atctgggcgg caaactcggg tggggccgag ccctggaacg tcgcgcaccc cagcgcacgt 2940

ttcatcatgc gcgtccacaa gaatgacacc attcaagtgt tcgattggga cgacagaaac 3000

aagtgtgtcg tggaaggaag caaccagatc aagaggatcg tacgcttgga accgagcgcc 3060

aacagaatgc ggctcgtcgg cataaacgac gcaggcaagt ttgacgaacg gcacaacgag 3120

ccaaacgatc ccttccgctg ggatcttgcc acgatcagca aactgaagct ccgccgcgcc 3180

cggcgcgtgc ggatcgacga attggggcgg gttcacacga tcccgcatgg gacagtttag 3240

## Description

### Field of the Invention

The invention relates to biotechnology, specifically to novel Cas nuclease enzymes of CRISPR-Cas systems, used for cutting DNA and editing the genome of various organisms. This technique may be used in the future for gene therapy of hereditary human diseases, as well as for editing the genome of other organisms.

### Background of the Invention

Modification of a DNA sequence is one of the topical problems in today's biotechnology field. Editing and modifying the genomes of eukaryotic and prokaryotic organisms, as well as manipulating DNA in vitro, require the targeted introduction of double-strand breaks in a DNA sequence.

To solve this problem, the following techniques are currently used: artificial nuclease systems containing domains of the zinc finger type, TALEN systems, and bacterial CRISPR-Cas systems. The first two techniques require laborious optimization of a nuclease amino acid sequence for recognition of a specific DNA sequence. In contrast, when it comes to CRISPR-Cas systems, the structures that recognize a DNA target are not proteins, but short guide RNAs. Cutting of a particular DNA target does not require the synthesis of nuclease or its gene de novo, but is made by way of using guide RNAs complementary to the target sequence. This makes CRISPR Cas systems convenient and efficient means for cutting various DNA sequences. The technique allows for simultaneous cutting of DNA at several regions using guide RNAs of different sequences. Such an approach is also used to simultaneously modify several genes in eukaryotic organisms.

By their nature, CRISPR-Cas systems are prokaryotic immune systems capable of highly specific introduction of breaks into a viral genetic material (Mojica F. J. M. et al. Intervening sequences of regularly spaced prokaryotic repeats derive from foreign genetic elements //Journal of molecular evolution. - 2005. - Vol. 60. - Issue 2. - pp. 174-182). The abbreviation CRISPR-Cas stands for "Clustered Regularly Interspaced Short Palindromic Repeats and CRISPR associated Genes" (Jansen R. et al. Identification of genes that are associated with DNA repeats in prokaryotes //Molecular microbiology. - 2002. - Vol. 43. - Issue 6. - pp. 1565-1575). All CRISPR-Cas systems consist of CRISPR cassettes and genes encoding various Cas proteins (Jansen R. et al., Molecular microbiology. - 2002. - Vol. 43. - Issue 6. - pp. 1565-1575). CRISPR cassettes consist of spacers, each having a unique nucleotide sequence, and repeated palindromic repeats (Jansen R. et al., Molecular microbiology. - 2002. - Vol. 43. - Issue 6. - pp. 1565-1575). The transcription of CRISPR cassettes followed by processing thereof results in the formation of guide crRNAs, which together with Cas proteins form an effector complex (Brouns S. J. J. et al. Small CRISPR RNAs guide antiviral defense in prokaryotes / / Science. - 2008. - Vol. 321. - Issue 5891. - pp. 960-964). Due to the complementary pairing between the crRNA and a target DNA site, which is called the protospacer, Cas nuclease recognizes a DNA target and highly specifically introduces a break therein.

CRISPR-Cas systems with a single effector protein are grouped into six different types (types I-VI), depending on Cas proteins that are included in the systems. The type II CRISPR-Cas9 system is characterized in its simple composition and mechanism of activity, i.e. its functioning requires the formation of an effector complex consisting only of one Cas9 protein and two short RNAs as follows: crRNA and tracer RNA (tracrRNA). The tracer RNA complementarily pairs with a crRNA region, originating from CRISPR repeat, to form a secondary structure necessary for the binding of guide RNAs to the Cas effector. The Cas9 effector protein is an RNA-dependent DNA endonuclease with two nuclease domains (HNH and RuvC) that introduce breaks into the complementary strands of target DNA, thus forming a double-strand DNA break (Deltcheva E. et al. CRISPR RNA maturation by trans-encoded small RNA and host factor RNase III //Nature. - 2011. - Vol. 471. - Issue 7340. - p. 602).

Thus far, several CRISPR-Cas nucleases are known that are capable of targeted and specific introduction of double-strand breaks into DNA. One of the main characteristics limiting the use of CRISPR-Cas systems is a PAM sequence that flanks a DNA target from the 3' end and the presence of which is necessary for the correct recognition of DNA by Cas9 nuclease. Various CRISPR-Cas proteins have different PAM sequences that limit the potential for use of the nucleases at any DNA regions. The use of CRISPR-Cas proteins with novel various PAM sequences is necessary to make it possible to modify any DNA region, both *in vitro* and in the genome of living organisms. Modification of eukaryotic genomes also requires the use of the small-sized nucleases to provide AAV-mediated delivery of CRISPR-Cas systems into cells.

Although a number of techniques for cutting DNA and modifying a genomic DNA sequence are known, there is still a need for novel effective means for modifying DNA in various organisms and at strictly specific sites of a DNA sequence. This invention provides a number of properties necessary for solving this problem.

### Summary of the invention

The object of the present invention is to provide novel means for modifying a genomic DNA sequence of unicellular or multicellular organisms using CRISPR-Cas9 systems. The current systems are of limited use due to a specific PAM sequence that must be present at the 3' end of a DNA region to be modified. Search for novel Cas9 enzymes with other PAM sequences will expand the range of available means for the formation of a double-strand break at desired, strictly specific sites in DNA molecules of various organisms. To solve this problem, the authors characterized the previously predicted type II CRISPR nuclease DfCas9 for *Defluviimonas sp.* 20V17, which can be used to introduce directed modifications into the genome of both the above and other organisms. The present invention is characterized in that it has the following essential features: (a) short PAM sequence that is different from other known PAM sequences; (b) relatively small size of the characterized DfCas9 protein, which is 1079 amino acid residues (a.a.r.).

Said problem is solved by using a protein comprising the amino acid sequence of SEQ ID NO: 1, or comprising an amino acid sequence that is at least 95% identical to the amino acid sequence of SEQ ID NO: 1 and differs from SEQ ID NO: 1 only in non-conserved amino acid residues, to form a double-strand break in a DNA molecule, located immediately before the nucleotide sequence 5'-NN(G/A)NA(C/T)N-3' in said DNA molecule. In some embodiments of the invention, this use is characterized in that the double-strand break is formed in a DNA molecule at a temperature of 35 °C to 37 °C and in the presence of Mn2+ ions. In preferred embodiments of the invention, this use is characterized in that the Mn2+ ion concentration is greater than 5 mM.

Said problem is further solved by providing a method for generating a double-strand break in a genomic DNA sequence of a unicellular or multicellular organism directly adjacent to the sequence 5'-NN(G/A)NA(C/T)N-3', comprising the introduction into at least one cell of said organism of an effective amount of: a) protein comprising the amino acid sequence of SEQ ID NO: 1, or a nucleic acid encoding the protein comprising the amino acid sequence of SEQ ID NO: 1, and b) guide RNA comprising a sequence that forms a duplex with the nucleotide sequence of an organism's genomic DNA region, which is directly adjacent to the nucleotide sequence 5'-NN(G/A)NA(C/T)N-3' and interacts with said protein following the formation of the duplex, or a DNA sequence encoding said guide RNA, wherein the interaction between said protein with the guide RNA and nucleotide sequence 5'-NN(G/A)NA(C/T)N-3' results in the formation of a double-strand break in the genomic DNA sequence directly adjacent to the sequence 5'-NN(G/A)NA(C/T)N-3' . In some embodiments of the invention, the method is characterized in that it further comprises the introduction of an exogenous DNA sequence simultaneously with the guide RNA.

A mixture of crRNA and tracer RNA (tracrRNA), which can form a complex with a target DNA region and DfCas9 protein, may be used as a guide RNA. In preferred embodiments of the invention, a hybrid RNA constructed based on crRNA and tracer RNA may be used as a guide RNA. Methods for constructing a hybrid guide RNA are known to those skilled (Hsu PD, et al., DNA targeting specificity of RNA-guided Cas9 nucleases. Nat Biotechnol. 2013 Sep;31(9):827-32). One of the approaches for constructing a hybrid RNA has been disclosed in the Examples below.

The invention may be used both for in vitro cutting target DNA, and for modifying the genome of some living organism. The genome may be modified in a direct fashion, i.e. by cutting the genome at a corresponding site, as well as by inserting an exogenous DNA sequence via homologous repair.

Any region of double-strand or single-strand DNA from the genome of an organism other than that used for administration (or a composition of such regions among themselves and with other DNA fragments) may be used as an exogenous DNA sequence, wherein said region (or composition of regions) is intended to be integrated into the site of a double-strand break in target DNA, induced by DfCas9 nuclease. In some embodiments of the invention, a region of double-strand DNA from the genome of an organism used for the introduction of DfCas9 protein, but further modified by mutations (substitution of nucleotides), as well as by insertions or deletions of one or more nucleotides, may be used as an exogenous DNA sequence.

The technical result of the present invention is to increase the versatility of the available CRISPR-Cas9 systems to enable the use of Cas9 nuclease for cutting genomic or plasmid DNA in a larger number of specific sites and specific conditions.

### Brief description of the drawings

Fig. 1. Scheme of CRISPR loci in *Defluviimonas sp.*
Fig. 2. Development of a system for cutting DNA limited to the sequence NN(G/A)NA(C/T)N.
Fig. 3. Scheme of interaction between the guide RNA and a region of target DNA.
Fig. 4. Dependence of DNA cutting reaction efficiency on the presence of ions.
Fig. 5. Checking of protein activity in cutting of various DNA targets.
Fig. 6. Reactions of in vitro cutting of a DNA fragment of the human grin2b gene.
Fig. 7. Effect of ion concentration on DNA cutting reaction efficiency.
Fig. 8. Selection of sgRNA sequence.
Fig. 9. Alignment of the initial portions of Cas9 protein sequences from Staphylococcus aureus (SaCas9), Campylobacter jejuni (CjCas9), and DfCas9 organisms. Non-conserved regions of sequences are underlined.

### Detailed disclosure of the invention

As used in the description of the present invention, the terms "includes" and "including" shall be interpreted to mean "includes, among other things". Said terms are not intended to be interpreted as "consists only of". Unless defined separately, the technical and scientific terms in this application have typical meanings generally accepted in the scientific and technical literature.

As used herein, the term "percent homology of two sequences" is equivalent to the term "percent identity of two sequences". The identity of sequences is determined based on a reference sequence. Algorithms for sequence analysis are known in the art, such as BLAST described in Altschul et al., J. Mol. Biol., 215, pp. 403-10 (1990). For the purposes of the present invention, to determine the level of identity and similarity between nucleotide sequences and amino acid sequences, the comparison of nucleotide and amino acid sequences may be used, which is performed by the BLAST software package provided by the National Center for Biotechnology Information (http://www.ncbi.nlm.nih.gov/blast) using gapped alignment with standard parameters. Percent identity of two sequences is determined by the number of positions of identical amino acids in these two sequences, taking into account the number of gaps and the length of each gap to be entered for optimal comparison of the two sequences by alignment Percent identity is equal to the number of identical amino acids at given positions taking account of sequence alignment divided by the total number of positions and multiplied by 100.

The term "specifically hybridizes" refers to the association between two single-strand nucleic acid molecules or sufficiently complementary sequences, which permits such hybridization under predetermined conditions typically used in the art.

The phrase "a double-strand break located **immediately** before the nucleotide PAM sequence" means that a double-strand break in a target DNA sequence will be made at a distance of 0 to 25 nucleotides before the nucleotide PAM sequence.

An exogenous DNA sequence introduced simultaneously with a guide RNA is intended to refer to a DNA sequence prepared specifically for the specific modification of a double-strand target DNA at the site of break determined by the specificity of the guide RNA. Such a modification may be, for example, an insertion or deletion of certain nucleotides at the site of a break in target DNA. The exogenous DNA may be either a DNA region from a different organism or a DNA region from the same organism as that of target DNA.

A protein comprising a specific amino acid sequence is intended to refer to a protein having an amino acid sequence composed of said amino acid sequence and possibly other sequences linked by peptide bonds to said amino acid sequence. An example of other sequences may be a nuclear localization signal (NLS), or other sequences that provide increased functionality for said amino acid sequence.

An exogenous DNA sequence introduced simultaneously with a guide RNA is intended to refer to a DNA sequence prepared specifically for the specific modification of a double-strand target DNA at the site of break determined by the specificity of the guide RNA. Such a modification may be, for example, an insertion or deletion of certain nucleotides at the site of a break in target DNA. The exogenous DNA may be either a DNA region from a different organism or a DNA region from the same organism as that of target DNA.

An effective amount of protein and RNA introduced into a cell is intended to refer to such an amount of protein and RNA that, when introduced into said cell, will be able to form a functional complex, i.e. a complex that will specifically bind to target DNA and produce therein a double-strand break at the site determined by the guide RNA and PAM sequence on DNA. The efficiency of this process may be assessed by analyzing target DNA isolated from said cell using conventional techniques known to those skilled.

A protein and RNA may be delivered to a cell by various techniques. For example, a protein may be delivered as a DNA plasmid that encodes a gene of this protein, as an mRNA for translation of this protein in cell cytoplasm, or as a ribonucleoprotein complex that includes this protein and a guide RNA. The delivery may be performed by various techniques known to those skilled.

The nucleic acid encoding system's components may be introduced into a cell directly or indirectly as follows: by way of transfection or transformation of cells by methods known to those skilled, by way of the use of a recombinant virus, by way of manipulations on the cell, such as DNA microinjection, etc.

A ribonucleic complex consisting of a nuclease and guide RNAs and exogenous DNA (if necessary) may be delivered by way of transfecting the complexes into a cell or by way of mechanically introducing the complex into a cell, for example, by way of microinjection.

A nucleic acid molecule encoding the protein to be introduced into a cell may be integrated into the chromosome or may be extrachromosomally replicating DNA. In some embodiments, to ensure effective expression of the protein gene with DNA introduced into a cell, it is necessary to modify the sequence of said DNA in accordance with the cell type in order to optimize the codons for expression, which is due to unequal frequencies of occurrence of synonymous codons in the coding regions of the genome of various organisms. Codon optimization is necessary to increase expression in animal, plant, fungal, or microorganism cells.

For a protein that has a sequence that is at least 95% identical to the amino acid sequence of SEQ ID NO: 1 to function in a eukaryotic cell, it is necessary for this protein to end up in the nucleus of this cell. Therefore, in some embodiments of the invention, a protein having a sequence that is at least 95% identical to the amino acid sequence of SEQ ID NO: 1 and which is further modified at one or both ends by the addition of one or more nuclear localization signals is used to form double-strand breaks in target DNA. For example, a nuclear localization signal from the SV40 virus may be used. To provide efficient delivery to the nucleus, the nuclear localization signal may be separated from the main protein sequence by a spacer sequence, for example, described in Shen B, et al. "Generation of gene-modified mice via Cas9/RNA- mediated gene targeting", Cell Res. 2013 May;23(5):720-3. Further, in other embodiments, a different nuclear localization signal or an alternative method for delivering said protein into the cell nucleus may be used.

The present invention encompasses the use of a protein from the *Defluviimonas sp.* 20V17 organism, which is homologous to the previously characterized Cas9 proteins, to introduce double-strand breaks into DNA molecules at strictly specified positions. The use of CRISPR nucleases to introduce targeted modifications to the genome has a number of advantages. First, the specificity of the system's activity is determined by a crRNA sequence, which allows for the use of one type of nuclease for all target loci. Secondly, the technique enables the delivery of several guide RNAs complementary to different gene targets into a cell at once, thereby making it possible to simultaneously modify several genes at once.

For the biochemical characterization of Cas9 protein from the Defluviimonas sp. 20V17 bacterium, the CRISPR locus encoding the main system components (genes of DfCas9, cas1, cas2 proteins, as well as CRISPR cassette and guide RNAs) was cloned into the single copy bacterial vector pACYC184. The effector ribonucleic complex consisting of Cas9 and a crRNA/tracrRNA duplex requires the presence of PAM (protospacer adjusted motif) on a DNA target for recognition and subsequent hydrolysis of DNA, in addition to crRNA spacer-protospacer complementarity. (Mojica F. J. M. et al. Short motif sequences determine the targets of the prokaryotic CRISPR defence system //Microbiology. - 2009. - Vol. 155. - Issue 3. - pp. 733-740). PAM is a strictly defined sequence of several nucleotides located in type II systems adjacent to or several nucleotides away from the 3' end of the protospacer on an off-target chain. In the absence of PAM, the hydrolysis of DNA bonds with the formation of a double-strand break does not take place. The need for the presence of a PAM sequence on a target increases recognition specificity, but at the same time imposes restraints on the selection of target DNA regions for introducing a break.

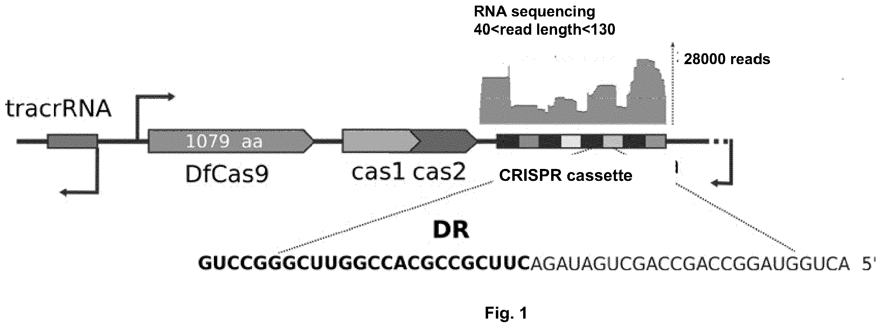

To determine the sequences of the guide RNA of the CRISPR-Cas9 system, RNA sequencing of *E.coli* DH5alpha bacteria bearing the generated DfCas9_pacyc184 construct was performed. The sequencing showed that the system's CRISPR cassette was actively transcribed, as was the tracer RNA (Fig. 1). Analysis of the crRNA and tracrRNA sequence allowed to contemplate that they may possibly form secondary structures recognized by DfCas9 nuclease.

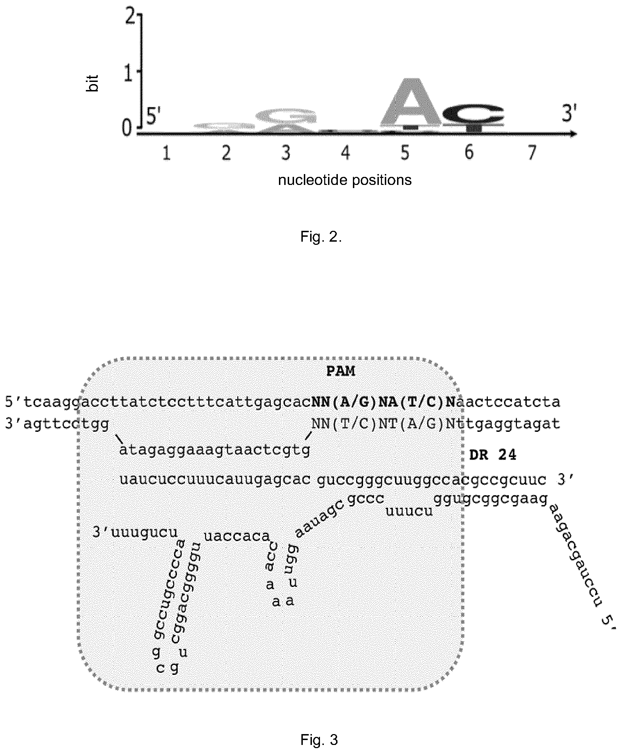

Further, the authors determined the PAM sequence of DfCas9 protein using bacterial PAM screening. In order to determine the PAM sequence of DfCas9 protein, *E.coli* DH5alpha cells bearing the DfCas9_pacyc184 plasmid were transformed by a plasmid library containing the spacer sequence 5'- TAGACCTTCGGGATCATGTCGATCATGATC-3' of CRISPR cassette of DfCas9 system flanked by a random seven-letter sequence from the 5' or 3' end. Plasmids bearing a sequence corresponding to the PAM sequence of DfCas9 system were subjected to degradation under the action of functional CRISPR-Cas system, whereas the remaining library plasmids were effectively transformed into cells, conferring them resistance to the antibiotic ampicillin. After transformation and incubation of the cells on plates containing the antibiotic, the colonies were washed off the agar surface, and DNA was extracted therefrom using the Qiagen Plasmid Purification Midi kit. Regions containing the randomized PAM sequence were amplified by PCR from the isolated pool of plasmids, and were then subjected to high-throughput sequencing on the Illumina platform. The resulting reads were analyzed by comparing the efficiency of transformation of plasmids with unique PAMs included in the library into cells bearing DfCas9_pacyc184 or into control cells bearing the empty vector pacyc184. The results were analyzed using bioinformatics methods. As a result, it was possible to identify the PAM of DfCas9 system, which was a three-letter sequence 5'-NN(G/A)NA(C/T)N-3' (Fig. 2).

To develop a system for cutting DNA *in vivo* and *in vitro,* it is necessary to obtain all the components that are part of the effector DfCas9 complex, as follows: guide RNAs and DfCas9 nuclease. Determination of the guide RNA sequence by RNA sequencing made it possible to synthesize crRNA and tracrRNA molecules in vitro. The synthesis was carried out using the NEB HiScribe T7 RNA synthesis kit.

To cut the DNA targets limited from the 3' end to the sequence NN(G/A)NA(C/T)N, the guide RNAs of the following sequence were used: tracrRNA 5'-UCCUAGCAGAAGAAGCGGCGUGGUCUUUCCCGCGAUAAGGUUAAAACCACAC CAUUGGGGCAGGCUGCGGCCUGCCCCAUCUGUUU3' and crRNA5' uaucuccuuucauugagcacGUCCGGGCUUGGCCACGCCGCUUC - 3'.

The first 20 nucleotides that are included in the crRNA sequence are complementarily paired with the corresponding sequence on the DNA target and provide specific recognition thereof by DfCas9 nuclease. If it is desired to cut a different DNA target, this 20-letter spacer sequence is modified (Fig. 3).

To obtain a recombinant DfCas9 protein, a gene thereof was cloned into the plasmid pET21a. E. coli Rosetta cells were transformed by the resulting plasmid DfCas9_pET21a. The cells bearing the plasmid were grown to an optical density of OD600=0.6, then the expression of DfCas9 gene was induced by adding IPTG to a concentration of 1mM. The cells were incubated for 4 hours at 25 °C, after which they were lyzed. The recombinant protein was purified in two stages as follows: by affinity chromatography (Ni-NTA) and by protein size-exclusion on a Superdex 200 column. The resulting protein was concentrated using Amicon 30 kDa filters. Thereafter, the protein was frozen at minus 80 °C and used for *in vitro* reactions.

As a target DNA, a double-strand DNA fragment with a length of 378 base pairs (bp) was used, bearing the protospacer sequence at a distance of about 30 bp from the 3' end, limited to the corresponding PAM sequence as determined in experiments on bacteria: NN(G/A)NA(C/T)N.

DNA target sequence: 5'-cccggggtaccacggagagatggtggaaatcatctttctcgtgggcatccttgatggccacctcgtcggaagtgccca cgaggatgacagcaatgccaatgctgggggggctcttctgagaacgagctctgctgcctgacacggccaggacggc caacaccaaccagaacttgggagaacagcactccgctctgggcttcatcttcaactcgtcgactccctgcaaacaca aagaaagagcatgttaaaataggatctacatcacgtaacctgtcttagaagaggctagatactgcaattcaaggacct tatctcctttcattgagcac**AAAAACG**aactccatctaccagcctactctcttatctctggtatt -3'.

The presence of divalent ions is required for the activity of nuclease domains of Cas9 proteins. In this regard, *in vitro* reactions on cutting the DNA target were carried out in the presence of magnesium, manganese, calcium and zinc salts. The *in vitro* reaction of cutting the DNA target was performed under the following conditions:
1x Tris-HCI buffer, pH = 7.9 (at 25 °C) 400 nM
DfCas9
20 nM DNA target
2 µM crRNA
2 µM tracrRNA
1 mM salt of the corresponding ion

The total reaction volume was 20 µl, the reaction duration was 30 minutes, and the incubation temperature was 37 °C.

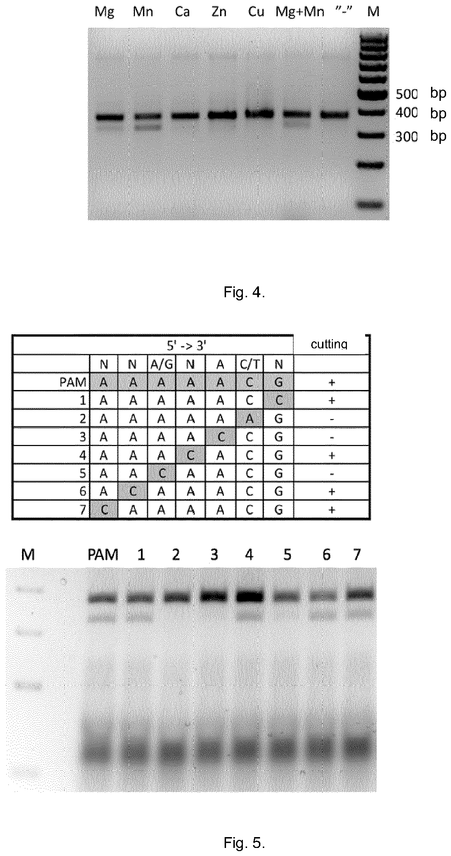

The reaction products were applied onto 1.5% agarose gel and subjected to electrophoresis. If cutting of DNA target was successful, the fragment broke up into two portions, one of which (about 325 bp long) formed a distinguishable band on the gel (Fig. 4). The results of experiments showed that DfCas9 protein requires, for activity thereof, manganese ions rather than magnesium ions, which is an unusual property for Cas9 nucleases characterized to date.

To confirm the significance of different DfCas9 PAM sequences, experiments were conducted on *in vitro* cutting of DNA targets, similar to those previously used, but comprising point (single-nucleotide) mutations in the PAM sequence 5'- AAAAACG -3' (Fig. 5).

The results of the experiment confirmed that DfCas9 enzyme is able to introduce double-strand breaks in DNA sequences limited to the PAM sequence NN(G/A)NA(C/T)N from the 3' end. Substitutions at PAM positions 3, 5, and 6 are critical for the DfCas9 effector complex and prevent effective cutting of target DNA.

In addition, experiments were conducted to find the optimal temperature for DfCas9 activity: it was found to be 35-37°C, which opens prospects for using DfCas9 in the human cells.

The following exemplary embodiments of the method are given for the purpose of disclosing the characteristics of the present invention and should not be construed as limiting in any way the scope of the invention.

### Example 1. Testing the activity of DfCas9 protein in the cutting of various DNA targets.

In order to test the ability of DfCas9 to recognize different DNA sequences flanked by the motif 5'-NN(G/A)NA(C/T)N-3', experiments were conducted in *in vitro* cutting of DNA targets from a human grin2b gene sequence (see Table 1).

**Table 1. DNA targets isolated from the human grin2b gene.**

| DNA target | PAM |
|---|---|
| attttctctcattctgcaga | gcaaata |
| tctaagacaggttacgtgat | gtagatc |
| aatgaaaggagataaggtcc | ttgaatt |
| caccttttattgccttgttc | aaggatt |
| ggcattgctgtcatcctcgt | gggcact |
| ttgcagtatctagcctcttc | taagaca |

The reactions of *in vitro* DNA cutting were performed under conditions similar to previous experiments. A guide crRNA was synthesized for each of the target sequences. A fragment of the human grin2b gene of about 760 bp in size was used as a DNA target: 5-ttgtctctgcctgtagctgccaatgactatagcaatagcaccttttattgccttgttcaaggatttctgaggcttttgaaagtttc attttctctcattctgcagagcaaataccagagataagagagtaggctggtagatggagttgggtttggtgctcaatgaa aggagataaggtccttgaattgcagtatctagcctcttctaagacaggttacgtgatgtagatcctattttaacatgctctttc tttgtgtttgcagggagtcgacgagttgaagatgaagcccagagcggagtgctgttctcccaagttctggttggtgttggc cgtcctggccgtgtcaggcagcagagctcgttctcagaagagcccccccagcattggcattgctgtcatcctcgtgggc acttccgacgaggtggccatcaaggatgcccacgagaaagatgatttccaccatctctccgtggtaccccggg - 3'.

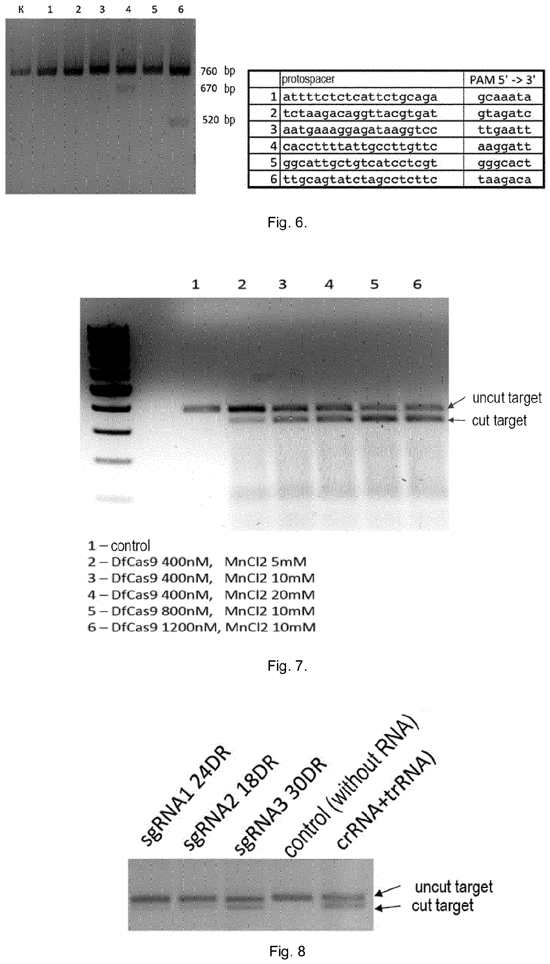

Sequences flanked from the 3' end by DNA regions corresponding to the PAM sequence 5'-NN(G/A)NA(C/T)N-3' were selected as DNA targets. Only two out of six selected sequences were effectively cut by DfCas9 protein: DNA fragments of the appropriate size cut by the effector complex are visible on the gel (Fig. 6). Selectivity in cutting different targets can be explained by the different efficiency of DfCas9 recognition of different secondary DNA structures, or other reasons. The DfCas9 selectivity in cutting different targets may increase the specificity of the protein when it cuts the genomes of eukaryotic cells.

Thus, DfCas9 in combination with guide RNAs is a novel means for cutting a double-strand DNA limited to the sequence 5'-NN(G/A)NA(C/T)N-3' with a characteristic temperature range of activity from 35 °C to 37 °C.

### Example 2. Effect of Mn2+ ion concentration on nuclease functionality.

To test the effect of Mn2+ ions on DfCas9 nuclease activity, experiments were performed *in vitro* in cutting a double-strand DNA fragment containing a target DNA sequence limited to the PAM sequence AAAAAC that has the consensus sequence 3'- NN(G/A)NA(C/T) -5'. The reaction was performed using a 1x CutSmart buffer (NEB), target DNA at a concentration of 20 nM, and trRNA/crRNA at a concentration of 2 µM.

As the DNA target, we used 5'-aataccagagataagagagtaggctggtagatggagttCGTTTTTgtgctcaatgaaaggagataaggtccttg aattgcagtatctagcctcttctaagacaggttacgtgatgtagatcctattttaacatgctctttctttgtgtttgcagggagtc gacgagttgaagatgaagcccagagcggagtgctgttctcccaagttctggttggtgttggccgtcctggccgtgtcag gcagcagagctcgttctcagaagagcccccccagcattggcattgctgtcatcctcgtgggcacttccgacgaggtgg ccatcaaggatgcccacgagaaagatgatttccaccatctctccgtggtaccccggg -3'.

The reaction used tracrRNA: 5'-UCCUAGCAGAAGAAGCGGCGUGGUCUUUCCCGCGAUAAGGUUAAAACCACAC CAUUGGGGCAGGCUGCGGCCUGCCCCAUCUGUUU-3' and crRNA: 5'-uaucuccuuucauugagcacGUCCGGGCUUGGCCACGCCGCUUC-3'.

Fig. 7 shows that an increase in the concentration of MnCl2 from 5 mM to 10 mM results in a more efficient cutting of the DNA target, whereas a further increase in the concentration of divalent ions does not influence the reaction efficiency. Thus, the effective DfCas9 activity requires the presence of manganese ions at a concentration of 10 mM or more.

### Example 3. Use of hybrid guide RNA for cutting a DNA target.

sgRNA is a form of guide RNAs, which is fused tracrRNA (tracer RNA) and crRNA. To select the optimal sgRNA, we constructed three variants of this sequence, which differed in the length of the tracrRNA-crRNA duplex. The RNAs were synthesized in vitro and experiments were performed on them in cutting the DNA target (Figure 8 shows the reactions of cutting DNA by DfCas9 using various sgRNA variants).

The selected sgRNA3 was just as effective as the native tracrRNA and crRNA sequences, with the cutting of more than 50% of the DNA target.

This variant of sgRNA may be used to cut any other target DNA when modifying a sequence that directly pairs with the DNA target.

The following RNA sequences were used as hybrid RNAs:
1-sgRNA1 24DR: 
UAUCUCCUUUCAUUGAGCACGUCCGGGCUUGGCCACGCCGCUUCGAAAGAA
GCGGCGUGGUCUUUCCCGCGAUAAGGUUAAAACCACACCAUUGGGGCAGGC
UGCGGCCUGCCCCAUCUGUUU
2 - sgRNA2 18DR 
UAUCUCCUUUCAUUGAGCACGUCCGGGCUUGGCCACGCGAAAGCGUG
GUCUUUCCCGCGAUAAGGUUAAACCACACCAUUGGGCAGGCUGCGGCCUG
CCCCAUCUGUUU
3 - sgRNA3 30DR 
**UAUCUCCUUUCAUUGAGCAC**GUCCGGGCUUGGCCACGCCGCUUCUUC
UGCGAAAGCAGAAGAAGCGGCGUGGUCUUUCCCGCGAUAAGGUUAAACCAC
ACCAUUGGGGCAGGCUGCGGCCUGCCCCAUCUGUUU
4 - control (without RNA)
5 - positive control (cutting the target using crRNA+trRNA)

Bold indicates a 20-nucleotide sequence that provides pairing with the target DNA (variable portion of sgRNA). The experiment used a control sample without RNA and a positive control, which is the cutting of the target using crRNA+trRNA.

The reaction was carried out under the following conditions: concentration of DNA sequence containing PAM (AAAACG) was 20 nM, protein concentration was 400 nM, RNA concentration was 2 µM; incubation time was 30 minutes, incubation temperature was 37 °C.

This hybrid RNA variant may be used to cut any other target DNA after modifying the sequence that directly pairs with the DNA target.

### Example 4. Cas9 proteins from closely related organisms belonging to Defluviimonas sp.

To date, no CRISPR-Cas9 enzymes have been characterized in *Defluviimonas.* Cas9 proteins from *Staphylococcus aureus,* that are comparable in size, are identical to DfCas9 by 21 %, Cas9 from *Campylobacter jejuni* are identical to DfCas9 by 28% (degree of identity was calculated using the BLASTp software, default parameters).

Thus, DfCas9 protein differs significantly in its amino acid sequence from other Cas9 proteins studied to date.

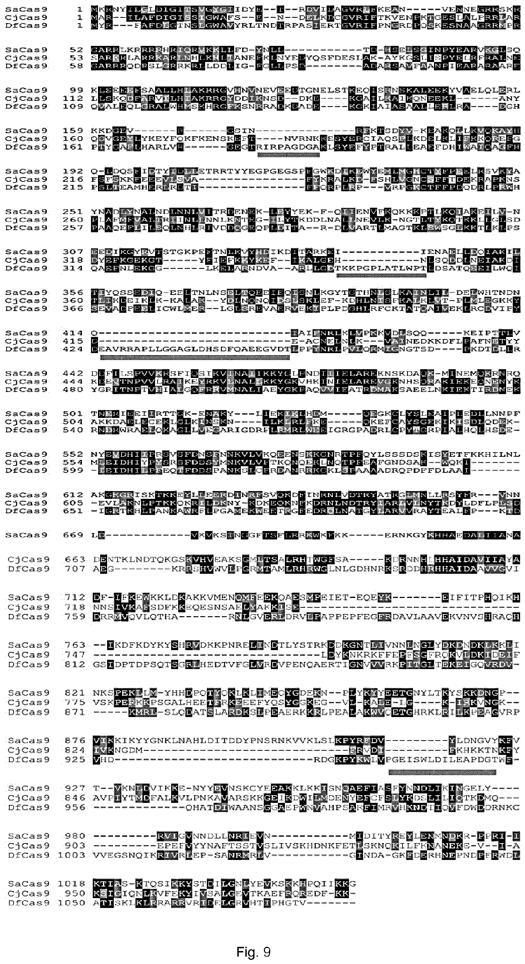

Those skilled in the art of genetic engineering will appreciate that DfCas9 protein sequence variant obtained and characterized by the Applicant may be modified without changing the function of the protein itself (for example, by directed mutagenesis of amino acid residues that do not directly influence the functional activity (Sambrook et al., Molecular Cloning: A Laboratory Manual, (1989), CSH Press, pp. 15.3-15.108)). In particular, those skilled will recognize that non-conserved amino acid residues may be modified, without affecting the residues that are responsible for protein functionality (determining protein function or structure). Examples of such modifications include the substitutions of non-conserved amino acid residues with homologous ones. Some of the regions containing non-conserved amino acid residues are shown in Figure 9. In some embodiments of the invention, it is possible to use a protein comprising an amino acid sequence that is at least 95% identical to the amino acid sequence of SEQ ID NO: 1 and differs from SEQ ID NO: 1 only in non-conserved amino acid residues, to form, in DNA molecule, a double-strand break located immediately before the nucleotide sequence 5'-NN(G/A)NA(C/T)N-3' in said DNA molecule. Homologous proteins may be obtained by mutagenesis (for example, site-directed or PCR-mediated mutagenesis) of corresponding nucleic acid molecules, followed by testing the encoded modified Cas9 protein for the preservation of its functions in accordance with the functional analyses described herein.

**Example 5.** DfCas9 system described in the present invention, in combination with guide RNAs, may be used to modify the genomic DNA sequence of a multicellular organism, including a eukaryotic organism. For introducing the DfCas9 system in the complex with guide RNAs into the cells of this organism (into all cells or into a portion of cells), various approaches known to those skilled may be applied. For example, methods for delivering CRISPR-Cas9 systems to the cells of organisms have been disclosed in the sources (Liu C et al., Delivery strategies of the CRISPR-Cas9 gene-editing system for therapeutic applications. J Control Release. 2017 Nov 28;266:17-26; Lino CA et al., Delivering CRISPR: a review of the challenges and approaches. Drug Deliv. 2018 Nov;25(1):1234-1257), and in the sources further disclosed within these sources.

For effective expression of DfCas9 nuclease in eukaryotic cells, it will be desirable to optimize codons for the amino acid sequence of DfCas9 protein by methods known to those skilled (for example, IDT codon optimization tool).

For the effective activity of DfCas9 nuclease in eukaryotic cells, it is necessary to import the protein into the nucleus of a eukaryotic cell. This may be done by way of using a nuclear localization signal from SV40 T-antigen (Lanford et al., Cell, 1986, 46: 575-582) linked to DfCas9 sequence via a spacer sequence described in Shen B, et al. "Generation of gene-modified mice via Cas9/RNA-mediated gene targeting", Cell Res. 2013 May;23(5):720-3, or without the spacer sequence. Thus, the complete amino acid sequence of nuclease to be transported inside the nucleus of a eukaryotic cell will be the following sequence: MAPKKKRKVGIHGVPAA-DfCas9- KRPAATKKAGQAKKKK (hereinafter referred to as DfCas9 NLS). A protein with the above amino acid sequence may be delivered using at least two approaches.

Gene delivery is accomplished by creating a plasmid bearing the DfCas9 NLS gene under control of a promoter (for example, CMV promoter) and a sequence encoding guide RNAs under control of the U6 promoter. As target DNAs, DNA sequences flanked by 3'- NN(G/A)NA(C/T)-5' are used, for example, sequences of the human grin2b gene:
5'-ttgcagtatctagcctcttc-3'
Thus, the crRNA expression cassette looks as follows:

Gagggcctatttcccatgattccttcatatttgcatatacgatacaaggctgttagagagataattggaattaatt
tgactgtaaacacaaagatattagtacaaaatacgtgacgtagaaagtaataatttcttgggtagtttgcagtttt
aaaattatgttttaaaatggactatcatatgcttaccgtaacttgaaagtatttcgatttcttggctttatatatcttgt
ggaaaggacgaaacaccgttgcagtatctagcctcttcGTCCGGGCTTGGCCACGCCGCTTCTT
CTGCTAGGATttttt

Bold indicates the U6 promoter sequence, followed by the sequence required for target DNA recognition, while the direct repeat sequence is highlighted in capital letters.

The tracer RNA expression cassette looks as follows:

Gagggcctatttcccatgattccttcatatttgcatatacgatacaaggctgttagagagataattggaattaatt
tgactgtaaacacaaagatattagtacaaaatacgtgacgtagaaagtaataatttcttgggtagtttgcagtttt
aaaattatgttttaaaatggactatcatatgcttaccgtaacttgaaagtatttcgatttcttggctttatatatcttgt
ggaaaggacgaaacaccgTCCTAGCAGAAGAAGCGGCGTGGTCTTTCCCGCGATAA
GGTTAAAACCACACCATTGGGGCAGGCTGCGGCCTGCCCCATCTGTTTtttt

Bold indicates the U6 promoter sequence, followed by the sequence encoding the tracer RNA. Plasmid DNA is purified and transfected into human HEK293 cells using Lipofectamine 2000 reagent (Thermo Fisher Scientific). The cells are incubated for 72 hours, after which genomic DNA is extracted therefrom using genomic DNA purification columns (Thermo Fisher Scientific). The target DNA site is analyzed by sequencing on the Illumina platform in order to determine the number of insertions/deletions in DNA that take place in the target site due to a directed double-strand break followed by repair thereof.

Amplification of the target fragments is performed using primers flanking the presumptive site of break introduction, for example, for the above-mentioned grin2b gene sites:
5'-GACTATAGCAATAGCAC-3'
5'TCAACTCGTCGACTCCCTG-3'

After amplification, samples are prepared according to the Ultra II DNA Library Prep Kit for Illumina (NEB) reagent sample preparation protocol for high-throughput sequencing. Sequencing is then performed on the Illumina platform, 300 cycles, direct reading. The sequencing results are analyzed by bioinformatic methods. An insertion or deletion of several nucleotides in the target DNA sequence is taken as a cut detection.

Delivery as a ribonucleic complex is carried out by incubating recombinant DfCas9 NLS with guide RNAs in the CutSmart buffer (NEB). The recombinant protein is produced from bacterial producer cells by purifying the former by affinity chromatography (NiNTA, Qiagen) with size exclusion (Superdex 200).

The protein is mixed with RNAs in a 1:2:2 ratio (DfCas9 NLS : crRNA: tracrRNA), the mixture is incubated for 10 minutes at room temperature and then transfected into cells.

Next, the DNA extracted therefrom is analyzed for insertions/deletions at the target DNA site (as described above).

The DfCas9 nuclease characterized in the present invention from the deep-sea bacterium *Defluviimonas sp. 20V17* has a number of advantages relative to the previously characterized Cas9 proteins. For example, DfCas9 has a relatively simple, three-letter PAM, distinct from other known Cas nucleases, that is required for the system to function. Most of the currently known Cas nucleases capable of introducing double-strand breaks in DNA have multi-letter complex PAMs that limit the choice of sequences suitable for cutting. Among the Cas nucleases studied to date, which recognize short PAMs, only DfCas9 can recognize sequences limited to 5'-NN(G/A)NA(C/T)N-3' nucleotides.

The second advantage of DfCas9 is a relatively small protein size (1079 a.a.r.). To date, it is one of the few small-sized proteins studied that have a simple PAM sequence and are active at 37 oC. An unusual property of DfCas9 is the need for the presence of manganese ions to successfully cut DNA targets. This property may be used to regulate the activity of the Cas9 complex.

Although the invention has been described with reference to the disclosed embodiments, those skilled in the art will appreciate that the particular embodiments described in detail have been provided for the purpose of illustrating the present invention and are not be construed as in any way limiting the scope of the invention. It will be understood that various modifications may be made without departing from the spirit of the present invention.

## Claims

1. Use of a protein comprising the amino acid sequence of SEQ ID NO: 1, or comprising an amino acid sequence that is at least 95% identical to the amino acid sequence of SEQ ID NO: 1 and differs from SEQ ID NO: 1 only in non-conserved amino acid residues, to form a double-strand break in a DNA molecule, located immediately before the nucleotide sequence 5'-NN(G/A)NA(C/T)N-3' in said DNA molecule.

2. The use according to Claim 1, **characterized in that** the double-strand break is formed in a DNA molecule at a temperature of 35 °C to 37 °C and in the presence of Mn2+ ions.

3. The use of the protein according to Claim 1, wherein the protein comprises the amino acid sequence of SEQ ID NO: 1.

4. A method for generating a double-strand break in a genomic DNA sequence of a unicellular or multicellular organism directly adjacent to the sequence 5'-NN(G/A)NA(C/T)N-3', comprising the introduction into at least one cell of said organism of an effective amount of: a) a protein comprising the amino acid sequence of SEQ ID NO: 1, or a nucleic acid encoding the protein comprising the amino acid sequence of SEQ ID NO: 1, and b) guide RNA comprising a sequence that forms a duplex with the nucleotide sequence of an organism's genomic DNA region, which is directly adjacent to the nucleotide sequence 5'-NN(G/A)NA(C/T)N-3' and interacts with said protein following the formation of the duplex, or a DNA sequence encoding said guide RNA,
wherein the interaction between said protein with the guide RNA and nucleotide sequence 5'-NN(G/A)NA(C/T)N-3' results in the formation of a double-strand break in the genomic DNA sequence directly adjacent to the sequence 5'-NN(G/A)NA(C/T)N-3' .

5. The method according to Claim 4 further comprising the introduction of an exogenous DNA sequence simultaneously with the guide RNA.
